# EUROPEAN PATENT SPECIFICATION

(11) **EP 0 681 477 B1**
(45) Date of publication and mention of the grant of the patent: **31.03.2004**
(21) Application number: 94907366.2
(22) Date of filing: 28.01.1994
(51) Int. Cl.: A61K 35/30, A61K 35/54, A61P 25/00

(54) **GENETIC MODIFICATION OF NEURAL STEM CELLS**
GENETISCHE MODIFIKATION VON NEURALEN STAMMZELLEN
MODIFICATION GENETIQUE DE CELLULES SOUCHES NERVEUSES

(30) Priority: 29.01.1993 US 10829
(43) Date of publication of application: 15.11.1995
(73) Proprietor: NEUROSPHERES HOLDINGS LTD., Calgary, Alberta T2N 4N1 (CA)
(72) Inventor: WEISS, Samuel, Calgary, Alberta T2L 1A6 (CA); REYNOLDS, Brent A., Calgary, Alberta T3B 2V6 (CA); HAMMANG, Joseph P., Barrington, RI 02806 (US); BAETGE, Edward E., Providence, RI 02903 (US)
(74) Representative: Baldock, Sharon Claire
(86) International application number: PCT/US1994/001053
(87) International publication number: WO 1994/016718

(56) References cited:
- WO-A-89/03872
- WO-A-90/06757
- WO-A-94/02593
- REYNOLDS B.A. AND WEISS S.: "Generation of neurons and astrocytes from isolated cells of the adult mammalian central nervous system" SCIENCE, vol. 255, no. 5052, 27 March 1992, pages 1707-1710, XP000561246
- GALIANA E. ET AL.: "Establishment of permanent astroglial cell lines, able to differentiate in vitro, from transgenic mice carrying the polyoma virus large T gene: an alternative approach to brain cell immortalization" JOURNAL OF NEUROSCIENCE RESEARCH, vol. 26, no. 1, May 1990, pages 269-277, XP000197501
- STEMPLE D.L. AND ANDERSON D.J.: "Isolation of a stem cell for neurons and glia from the mammalian neural crest" CELL, vol. 71, 11 December 1992, pages 973-985, XP002063900
- MARVIN M. AND MCKAY R.: "Multipotential stem cells in the vertebrate CNS" SEMINARS IN CELL BIOLOGY, vol. 3, 1992, pages 401-411, XP002064929
- GAGE F.H. AND FISHER L.J.: "Intracerebral grafting: a tool for the neurobiologist" NEURON, vol. 6, January 1991, pages 1-12, XP002064991
- VRARD C. ET AL.: "Transfert de gènes dans les cellules nerveuses. Immortalisation cellulaire et marquage génétique" M/S MEDICINE SCIENCES, vol. 7, no. 4, April 1991, pages RIX-RXII, XP002064930
- RENFRANZ P.J. ET AL.: "Region-specific differentiation of the hippocampal stem cell line HiB5 upon implantation into the developing mammalian brain" CELL, vol. 66, 23 August 1991, pages 713-729, XP002064931
- SNYDER E.Y. ET AL.: "Multipotent neural cell lines can engraft and participate in development of mouse cerebellum" CELL, vol. 68, 10 January 1992, pages 33-51, XP002064932
- HAMMANG J.P. ET AL.: "EGF-responsive neural stem cells derived from MBP-lacZ transgenic mice can express the transgene in differentiating oligodendrocytes" MOLECULAR BIOLOGY OF THE CELL, vol. 4, no. suppl., 1993, page 374a XP002064933
- Annual Review of Neuroscience, Volume 12, issued 1989, C. CEPKO, "Immortalization of Neural Cells via Retrovirus-Mediated Oncogene Transduction", pages 47-65, see entire article.
- Science, Volume 241, issued 23 September 1988, GELLER et al., "A Defective HSV-1 Vector Expresses Escherichia Coli, Beta-Galactosidase in Cultured Peripheral Neurons", pages 1667-1669, see entire article.
- Neuron, Volume 1, issued August 1988, FREDERIKSEN et al., "Immortalization of Precursor Cells from the Mammalian CNS", pages 439-448, see entire article.
- Society for Neurosciences, Volume 16, issued 1990, REYNOLDS et al., "EGF- and TGF-alpha Responsive Striatal Embryonic Progenitor Cells Produce both Neurons and Astrocytes", page 1147, column 2, Abstract No. 474.2.
- Developmental Brain Research, Volume 54, issued 1990, HUNTR et al., "Growth Factor Responses of Enriched Biopotential Glial Progenitors", pages 235-248, see entire article.
- Nature, Volume 347, issued 25 October 1990, CATTANEO et al., "Proliferation and Differentiation of Neuronal Stem Cells Regulated by Nerve Growth Factor", pages 762-765, see entire article.
- Science, Volume 242, issued 16 December 1988, ROSENBERG et al., "Grafting Genetically Modified Cells to the Damaged Brain: Restorative Effects of NGF Expression", pages 1575-1578, see entire article.
- Proceedings of the National Academy of Sciences, Volume 86, issued November 1989, WOLFF et al., "Grafting Fibroblasts Genetically Modified to Produce L-Dopa in a Rat Model of Parkinson Disease", pages 9011-9014, see entire article.

## Description

### BACKGROUND OF THE INVENTION

The present invention is directed to methods of producing genetically modified cells useful in the treatment of central nervous system (CNS) disorders. More particularly, the present invention relates to the genetic modification of growth factor responsive neural stem cells and of differentiated neuronal and glial cells derived from growth factor-responsive neural stem cells and the use of these cells for the treatment of neurodegenerative disorders.

CNS disorders encompass numerous afflictions such as neurodegenerative diseases (e.g. Alzheimer's and Parkinson's), acute brain injury (e.g. stroke, head injury, cerebral palsy) and a large number of CNS dysfunctions (e.g. depression, epilepsy, and schizophrenia). In recent years neurodegenerative disease has become an important concern due to the expanding elderly population which is at greatest risk for these disorders. These diseases, which include Alzheimer's Disease, Multiple Sclerosis, Huntington's Disease, Amyotrophic Lateral Sclerosis, and Parkinson's Disease, have been linked to the degeneration of neural cells in particular locations of the CNS, leading to the inability of these cells or the brain region to carry out their intended function. In addition to neurodegenerative diseases, acute brain injuries often result in the loss of neural cells, the inappropriate functioning of the affected brain region, and subsequent behavior abnormalities. Probably the largest area of CNS dysfunction (with respect to the number of affected people) is not characterized by a loss of neural cells but rather by an abnormal functioning of existing neural cells. This may be due to inappropriate firing of neurons, or the abnormal synthesis, release, and processing of neurotransmitters. These dysfunctions may be the result of well studied and characterized disorders such as depression and epilepsy, or less understood disorders such as neurosis and psychosis.

To date, treatment for CNS disorder has been primarily via the administration of pharmaceutical compounds. Unfortunately, this type of treatment has been fraught with many complications including the limited ability to transport drugs across the blood-brain barrier and the drug-tolerance which is acquired by patients to whom these drugs are administered long-term. For instance, partial restoration of dopaminergic activity in Parkinson's patients has been achieved with levodopa, which is a dopamine precursor able to cross the blood-brain barrier. However, patients become tolerant to the effects of levodopa, and therefore, steadily increasing dosages are needed to maintain its effects. In addition, there are a number of side effects associated with levodopa such as increased and uncontrollable movement.

Recently, the concept of neurological tissue grafting has been applied to the treatment of neurodegenerative diseases such as Parkinson's Disease. Neural grafts may avert the need not only for constant drug administration, but also for complicated drug delivery systems which arise due to the blood-brain barrier.

Genetically modified cells have been used in neurological tissue grafting in order to replace lost cells which normally produce a neurotransmitter. For example, fibroblasts have been genetically modified with a retroviral vector containing a cDNA for tyrosine hydroxylase, which allows them to produce dopamine, and implanted into animal models of Parkinson's Disease (Gage at al., US Patent No. 5,082,670).

While the use of genetically modified fibroblasts to treat CNS disorders has shown promise in improving some behavioral deficits in animal models of Parkinson's Disease, and represents a novel approach to supplying a needed transmitter to the CNS, it suffers from several significant drawbacks as a treatment for Parkinson's Disease and in general as a therapeutic approach for treating neurodegenerative diseases and brain injury. First, the CNS is primarily composed of three cell types -- neurons, astrocytes and oligodendrocytes. Fibroblasts are not present. The implantation of a foreign cell in the CNS and its direct and indirect effects on the functioning of the host cells has yet to be studied. However, it is likely that the expression of membrane bound factors and the release of soluble molecules such as growth factors and proteases will alter the normal behavior of the surrounding tissue. This may result in the disruption of neuronal firing patterns either by a direct action on neurons or by an alteration in the normal functioning of glial cells.

Another concern that arises when fibroblasts are implanted into the CNS is the possibility that the implanted cells may lead to tumor formation because the intrinsic inhibition of fibroblast division is poorly controlled. Instead, extrinsic signals play a major role in controlling the number of divisions the cell will undergo. The effect of the CNS environment on the division of implanted fibroblasts and the high probability of a fibroblastic tumor formation has not been studied in the long-term.

A third concern in transplanting fibroblasts into the CNS is that fibroblasts are unable to integrate with the CNS cells as astrocytes, oligodendrocytes, or neurons do. Fibroblasts are intrinsically limited in their ability to extend neuronal-like processes and form synapses with host tissue. Hence, although the genetic modification and implantation of fibroblasts into the CNS represents an improvement over the current technology for the delivery of certain molecules to the CNS, the inability of fibroblasts to integrate and function as CNS tissue, their potential negative effects on CNS cells, and their limited intrinsic control of proliferation limits their practical usage for implantation for the treatment of acute or chronic CNS injury or disease.

A preferred tissue for genetic modification and implantation would be CNS cells -- neurons, astrocytes, or oligodendrocytes. One source of CNS cells is from human fetal tissue. Several studies have shown improvements in patients with Parkinson's Disease after receiving implants of fetal CNS tissue. Implants of embryonic mesencephalic tissue containing dopamine cells into the caudate and putamen of human patients was shown by Freed et al (N Engl J Med 327:1549-1555 (1992)) to offer long-term clinical benefit to some patients with advanced Parkinson's Disease. Similar success was shown by Spencer et al (N Engl J Med 327:1541-1548 (1992)). Widner et al (N Engl J Med 327:1556-1563 (1992)) have shown long-term functional improvements in patients with MPTP-induced Parkinsonism that received bilateral implantation of fetal mesencephalic tissue.

While the studies noted above are encouraging, the use of aborted fetal tissue for the treatment of disease raises ethical considerations and political obstacles. There are other considerations as well. Fetal CNS tissue is composed of more than one cell type, and thus is not a well-defined source of tissue. In addition, there are serious doubts as to whether an adequate and constant supply of fetal tissue would be available for transplantation. For example, in the treatment of MPTP-induced Parkinsonism (Widner *supra*) tissue from 6 to 8 fetuses were used for implantation into the brain of a single patient. There is also the added problem of the potential for contamination during fetal tissue preparation. Moreover, the tissue may already be infected with a bacteria or virus, thus requiring expensive diagnostic testing for each fetus used. However, even diagnostic testing might not uncover all infected tissue. For example, the diagnosis of HIV-free tissue is not guaranteed because antibodies to the virus are generally not present until several weeks after infection.

Instead of fetal tissue, it would be more preferable to have a well-defined, reproducible source of tissue for transplantation that is available in unlimited amounts. Since neural tissue undergoes minimal division, it does not readily meet these criteria. While astrocytes retain the ability to divide and are probably amenable to infection with foreign genes; their ability to form synapses with neuronal cells is limited and consequently so is their extrinsic regulation of the expression and release of the foreign gene product.

Oligodendrocytes suffer from some of the same problems. In addition, mature oligodendrocytes do not divide, limiting the infection of oligodendrocytes to their progenitor cells (i.e. 02A cells). However, due to the limited proliferative ability of oligodendrocyte progenitors, the infection and harvesting of these cells does not represent a practical source.

The infection of neurons with foreign genes and implantation into the CNS would be ideal due to their ability to extend processes, make synapses and be regulated by the environment. However, differentiated ne'urons do not divide and transfection with foreign genes by chemical and physical means is not efficient, nor are they stable for long periods of time. The infection of primary neuronal precursors with retroviral vectors *in vitro* is not practical either because neuroblasts are intrinsically controlled to undergo a limited number of divisions making the selection of a large number of neurons, that incorporate and express the foreign gene, nearly impossible. There are a number of reports of immortalizing neural cells with oncogenes, for example, see Renfranz et al (Cell, 1991, Vol. 6, pp 713-729), Snyder et al (Cell, 1992, Vol. 68, pp 33-51), Galiana et al (Journal of Neuroscience Research, 1990, Vol. 26, pp 269-277), Constance L. Cepho (Ann, Rev. Neurosci. 1989, Vol. 12, pp 47-65) and WO89/03872. The possibility of immortalizing the neuronal precursors by retroviral transfer of oncogenes and their subsequent infection of a desired gene is not preferred due to the potential for tumor formation by the implanted cells.

The growth of non-immortalized striatal cells isolated from embryonic and adult mouse brain in the presence of growth factors is described in Reynolds et al in Society for Neurosciences, 16 1147 Co 2, abstract No. 474.2 and Science, 1992, Vol.255, pp 1707-1710.

One attempt of overcoming the problems associated with the inability of neurons to undergo a significant number of divisions is disclosed by Ronnett et al. who have established a neuronal cell line (Science, 248: 603-605 (1990)). The cell line is non-malignant, but is derived from cerebral cortical tissue obtained from a patient with unilateral megalencephaly, a low-grade proliferation and migration disorder of neurons. However, given the characteristics of this cell line, it is questionable as to whether the cells would be capable of stopping division once implanted into a host CNS.

Another approach to introducing foreign genes into the CNS is suggested by Morshead and Van Der Koy, (J. of Neurosci, 12(1): 249-256 (1992)). Subependymal cells were infected with replication-deficient recombinant retrovirus carrying the gene for *E-coli* β-galactosidase. It was found using ³H-thymidine and BrdU incorporation that there is continuous mitotic division in this region of the CNS. Morshead and Van Der Koy suggest that the proliferating cells are stem cells based on the fact that the cells continuously divide in an asymmetrical stem cell fashion; that is, the dividing cell gives rise to one multipotential cell while the fate of the other daughter is cell death. The basis for this idea is that while there is a constant rate of division,there is no relative increase in cell number in that region of CNS. Transfection of primary rat cerebellar cultures with retrovirus carrying the *E-coli* Lac Z gene is also described by McKay et al (Cold Spring Harbour Symposia on quantitative Biology, 1990, Vol. LV, Cold Spring Laboratory Press). A similar approach has been taken by Geller and Breakfield (1988) Vol. 241, pp 1667-1669 were adult neurons are transfected with a defective herpes simplex virus carrying the *E-coli* Lac Z gene.

The fact that the endogenous stem cells continuously divide makes them suitable targets for genetic modification via retroviral infection. However, these cells exist within the CNS at a density of less than about 5 cells/100 cubic microns and their rate and number of divisions is likely to be at least partially under epigenetic control. Thus a major limitation of the endogenous proliferating subependymal stem cells is that they are present in the CNS in a fixed number and the introduction of new genes would be difficult to control. A second major limitation is that in rodents and primates the cells appear to be spatially restricted to the subependymal layer and neither the stem cell nor its progeny migrate to other regions of the CNS (Smart, *J. of Comp. Neurology*, 116:325-347 (1961); Lewis, *Nature,* 217:974-975, (1968)). Similar cells have not been described in other CNS regions and if they do exist, they are not characterized.

The inability in the prior art of non-CNS tissue transplants to fully integrate into the host tissue, and the lack of availability of non-tumorigenic, integratable CNS cells in unlimited amounts from a reliable source for grafting are, perhaps, the greatest limitations of neurotransplantation.

### SUMMARY OF THE INVENTION

In view of the aforementioned deficiencies attendant with prior art, including methods of neural cell culturing and transplantation, it should be apparent that there still exists a need in the art for a reliable source of unlimited numbers of non-transformed cells for neurotransplantation, that can be genetically modified and that are capable of differentiating into neurons and glial cells, and capable of integrating into the mammalian CNS.

Accordingly, it is an object of this invention to provide a reliable source of genetically modified and epigenetically regulated cells for transplantation, which, upon transplantation into the CNS, further differentiate into neurons and glial cells and integrate into the existing CNS structure.

It is another object of this invention to provide an abundant source of cells that are highly manipulatable for the purpose of genetic modification.

It is yet another object of this invention to provide genetically modified cells which can be readily implanted nearly anywhere in the CNS.

These and other objects and features of the invention will be apparent to those skilled in the art from the following detailed descriptions and appended claims.

A method of producing genetically modified cells useful in the treatment of CNS disorders is disclosed. The method comprises the steps of (a) isolating neural stem cells from donor tissue, (b) proliferating the neural stem cells in a culture medium containing one or more growth factors to obtain precursor cells, and (c) genetically modifying the stem and precursor cells to produce cells that are useful in the treatment of CNS disorders.

In particular, the invention relates to a method of producing non-tumorigenic, genetically modified multipotent central nervous system (CNS) neural stem cells, comprising the steps of:
(a) isolating multipotent CNS neural stem cells from neural tissue to the exclusion of human embryonic tissue/cells, wherein the neural stem cells have not been immortalised with an oncogene, said CNS neural stem cells being capable of producing progeny that are capable of differentiating into neurons, astrocytes, and oligodendrocytes;
(b) proliferating said multipotent CNS neural stem cells in a serum-free culture medium containing a growth factor effective for inducing multipotent CNS neural stem cell proliferation,
(c) further propagating the proliferated cells, and
(d) genetically modifying said propagated cells, by introducing an exogenous polynucleotide to the proliferated cells.

The non-tumorigenic, genetically modified CNS neural stem cells of the invention are useful in a method of treating CNS disorders whereby genetically modified precursor cells that produce a biologically active substance useful for the treatment of a CNS disorder are transplanted into CNS of a recipient.

None of the foregoing references is believed to disclose the present invention as claimed and is not presumed to be prior art. The references are offered for the purpose of background information.

### BRIEF DESCRIPTION OF THE DRAWINGS

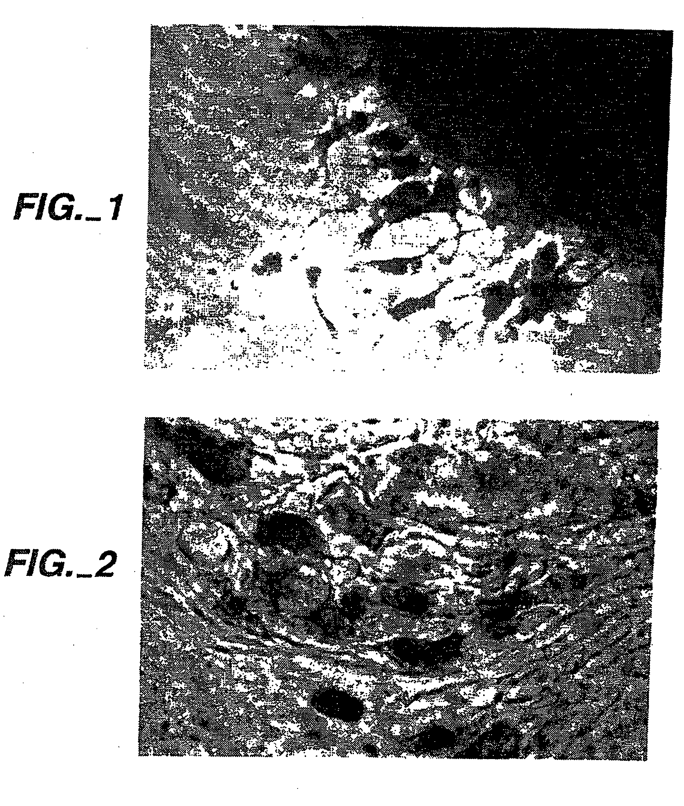

FIG. 1: A photograph of genetically modified precursor cells infected with a retrovirus containing the β-galactosidase gene. Cells containing the gene exhibit a characteristic blue reaction product.
FIG. 2: A photograph of a 2-4 week post implantation section of newborn mouse cortex into which β-galactosidase-containing precursor cells were implanted. Implanted cells containing the β-galactosidase gene exhibit a characteristic blue reaction product.

### DESCRIPTION OF THE PREFERRED EMBODIMENTS

Neural stem cells (NSCs) have been reported and their potential use has been described by Reynolds and Weiss, Science 255:1707 (1992). The term "stem cell" refers to an undifferentiated cell which is capable of unlimited proliferation and giving rise to more stem cells having the ability to generate a large number of progenitor cells that can in turn give rise to differentiated, or differentiable daughter cells. The term "neural stem cell" (NSC) refers to the stem cells of the instant invention, the progeny of which under appropriate culturing conditions, include both glial and neuronal progenitor cells. It has been shown that NSCs give rise to neuroblasts (Reynolds and Weiss, Restorative Neurology & Neuroscience 4:208 (1992). It is also known that NSCs give rise to major macroglial cell types (astrocytes and oligodendrocytes).

Neural stem cells can be generated from a variety of donor tissues including adult or fetal neural tissue from human or other animal sources, and cultured by the method of Reynolds and Weiss (*supra*). The term "donor" refers to the human or other animal which is the source of the neural stem cells used in the instant invention. In some situations, the donor will be a transgenic animal. The term "transgenic animal" refers to non-human animals in which a segment of DNA has been physically integrated into the genome of all cells, including germ cells, so that the DNA can be transmitted to offspring.

The neural stem cells of the present invention are growth factor-responsive. When grown in a defined serum-free medium, and in the presence of a growth factor or mitogen such as epidermal growth factor (EGF) or the like, the cells are induced to divide giving rise to clusters of undifferentiated cells. The term "neurosphere" is used to refer to the cluster of cells. At least some of the cells are of the nestin (+) phenotype. The cluster is comprised of stem cells and/or progenitor cells and may or may not include differentiated cells. The term "progenitor cells" refers to undifferentiated cells derived from neural stem cells, the progeny of which may, under appropriate conditions, include glial and/or neuronal progenitor cells.

As used herein, the term "precursor cells" refers to the living cells of the instant invention that are derived from neural stem cells proliferated in a culture medium containing a growth factor or combination of growth factors, and includes both progenitor and stem cells. The cells of the neurosphere are hereinafter referred to as precursor cells. Precursor cells are typically grown in the form of neurospheres, but may exhibit different growth patterns depending upon culture conditions. The term "growth factor" as used herein, refers to a protein, peptide, mitogen, or other molecule having a growth, proliferative, differentiative, or trophic effect. The term growth factor also encompasses any combination of growth factors. Such growth factors include nerve growth factor (NGF), acidic and basic fibroblast growth factor (aFGF, bFGF), platelet-derived growth factor (PDGF), thyrotropin-reteasing hormone (TRH), EGF, an EGF-like ligand, amphiregulin, transforming growth factor alpha and beta (TGFα, TGFβ), insulin-like growth factors (IGFs), and the like.

Although the precursor cells are non-transformed primary cells, they possess features of a continuous cell line. In the undifferentiated state in the presence of a growth factor such as EGF, the cells continuously divide and are therefore excellent targets for genetic modification. The term "genetic modification" as used herein refers to the stable or transient alteration of the genotype of a precursor cell by introduction of exogenous DNA. DNA may be synthetic, or naturally derived, and may contain genes, portions of genes, or other useful DNA sequences. The term "genetic modification" as used herein is not meant to include naturally occurring alterations such as that which occurs through natural viral activity, natural genetic recombination, or the like. Exogenous DNA may be introduced to a precursor cell by viral vectors (retrovirus, modified herpes viral, herpes-viral, adenovirus, adeno-associated virus, and the like) or direct DNA transfection (lipofection, calcium phosphate transfection, DEAE-dextran, electroporation, and the like). The genetically modified cells of the present invention possess the added advantage of having the capacity to fully differentiate to produce neurons or macroglial cells in a reproducible fashion using a number of differentiation protocols.

Precursor cells can be derived from non-human transgenic animals, and thus are in a sense already genetically modified. There are several methods presently used for generating transgenic animals. The technique used most often is direct microinjection of DNA into single-celled fertilized eggs. Other techniques include retroviral-mediated transfer, or gene transfer in embryonic stem cells. These techniques and others are detailed by Hogan et al. in *Manipulating the Mouse Embryo, A Laboratory Manual* (Cold Spring Harbor Laboratory Ed., 1986), incorporated herein by reference. Use of these transgenic animals has certain advantages including the fact that there is no need to transfect healthy neurospheres. Precursor cells derived from transgenic animals will exhibit stable gene expression. Using transgenic animals, it is possible to breed in new genetic combinations. The transgenic animal may have integrated into its genome any useful gene that is expressed by neural cells. Examples of useful DNA are given below in the discussion of genetically modifying precursor cells.

A significant challenge for cellular transplantation in the CNS is the identification of the donor cells after implantation within the host. A number of strategies have been employed to mark donor cells, including tritiated labels, fluorescent dyes, dextrans, and viral vectors carrying reporter genes. However, these methods suffer from inherent problems of toxicity, stability, or dilution over the long term. The use of neural cells derived from transgenic animals may provide an improved means by which identification of transplanted neural cells can be achieved. A transgenic marking system provides a more stable and efficient method for cell labeling. In this system, promoter elements, for example for glial fibrillary acidic protein (GFAP) and myelin basic protein (MBP), can direct the expression of the *E. coli* β-galactosidase reporter gene in transgenic mice. In these systems, cell-specific expression of the reporter gene occurs in astrocytes (GFAP-*lacZ*) and in oligodendrocytes (MBP*-lacZ*) in a developmentally-regulated manner.

Once propagated, the neurosphere cells are mechanically dissociated into a single cell suspension and plated on petri dishes in a medium where they are allowed to attach overnight. The precursor cells are then genetically modified. If the precursor cells are generated from transgenic animals, then they may or may not be subjected to further genetic modification, depending upon the properties desired of the cells. Any useful genetic modification of the cells is within the scope of the present invention. For example, precursor cells may be modified to produce or increase production of a biologically active substance such as a neurotransmitter or growth factor or the like. The genetic modification is performed either by infection with recombinant retroviruses or transfection using methods known in the art (see Maniatis et al., in *Molecular Cloning: A Laboratory Manual,* Cold Spring Harbor Laboratory, N.Y. (1982)). Briefly, the chimeric gene constructs will contain viral, for example retroviral long terminal repeat (LTR), simian virus 40 (SV40), cytomegalovirus (CMV); or mammalian cell-specific promoters such as tyrosine hydroxylase (TH), dopamine β-hydroxylase (DBH), phenylethanolamine N-methyltransferase (PNMT), choline acetyltransferase (ChAT), glial fibrillary acidic protein (GFAP), neuron-specific enolase (NSE), the neurofilament (NF) proteins (NF-L, NF-M, NF-H, and the like) that direct the expression of the structural genes encoding the desired protein. In addition, the vectors will include a drug selection marker, such as the *E. coli* aminoglycoside phosphotransferase gene, which when coinfected with the experimental gene confers resistance to geneticin (G418), a protein synthesis inhibitor.

When the genetic modification is for the production of a biologically active substance, the substance will generally be one that is useful for the treatment of a given CNS disorder. For example, it may be desired to genetically modify cells so they secrete a certain growth factor product. As used herein, the term "growth factor product" refers to a protein, peptide, mitogen, or other molecule having a growth, proliferative, differentiative, or trophic effect. Growth factor products useful in the treatment of CNS disorders include, but are not limited to, nerve growth factor (NGF), brain-derived neurotrophic factor (BDNF), the neuroirophins (NT-3, NT-4/NT-5), ciliary neurotrophic factor (CNTF), amphiregulin, bFGF, aFGF, EGF, TGFα, TGFβ, PDGF, IGFs, and the interleukins.

Cells can also be modified to express a certain growth factor receptor (r) including, but not limited to, p75 low affinity NGFr, CNTFr, the *trk* family of neurotrophin receptors (*trk, trk*B, *trk*C), EGFr, FGFr, and amphiregulin receptors. Cells can be engineered to produce various neurotransmitters or their receptors such as serotonin, L-dopa, dopamine, norepinephrine, epinephrine, tachykinin, substance-P, endorphin, enkephalin, histamine, N-methyl D-aspartate (NMDA), glycine, glutamate, γ-amino butyric acid (GABA), acetylcholine, and the like. Useful neurotransmitter-synthesizing genes include TH, dopa decarboxylase (DDC), DBH, PNMT, glutamic acid decarboxylase (GAD), tryptophan hydroxylase, ChAT, and histidine decarboxylase. Genes that encode for various neuropeptides, which may prove useful in the treatment of CNS disorders, include substance-P, neuropeptide-Y (NP-Y), enkephalin, vasopressin, vasoactive intestinal peptide (VIP), glucagon, bombesin, cholecystokinin (CCK), somatostatin, calcitonin gene-related peptide (CGRP), and the like.

After successfully transfected/infected cells are selected they are cloned using limiting dilution in 96 multi-well plates and assayed for the presence of the desired biologically active substance. Clones that express high levels of the desired substance are grown and their numbers expanded in T-flasks. The specific cell line can then be cyropreserved. The genetically modified precursor cell line can be used for cell/gene therapy, and thus implanted into the CNS of a recipient in need of the biologically active molecule produced by the genetically modified cells. Multiple clones of genetically modified precursor cells will be obtained. Some may give rise preferentially to neuronal cells, and some to glial cells.

Alternatively, the genetically modified precursor cells can be subjected to various differentiation protocols *in vitro* prior to implantation. For example, genetically modified precursor cells may be removed from the culture medium which allows proliferation and treated with a medium containing a growth factor or combination of growth factors that induces the differentiation of the cells into neurons or macroglial cells. The differentiation protocols are set forth in co-pending patent application U.S.S.N. 07/967,622, filed 28 October 1992 and published as WO 94/10292 on 11.5.1994. The protocol used will depend upon the type of genetically modified cell desired. Differentiation can also be induced by plating the precursor cells on a fixed substrate, including poly-L-ornithine or poly-L-lysine coated flasks or the like.

Various markers are used to identify the differentiated cells. Oligodendrocytes precursor cells, which give rise to oligodendrocytes can have the phenotype A2B5 (+), 04( + )/GaIC(-), MBP(-) and GFAP(-) [but are not limited to this phenotype]. Oligodendrocytes, which are glial cells that form the myelin surrounding axons in the CNS, have the phenotype galactocerebroside (+), myelin basic protein (+), and glial fibrillary acidic protein (-) [GalC(+), MBP( +), GFAP(-)].

Neurons may possess one or more of the following phenotypic markers: neuron-specific enolase (NSE), the neurofilament (NF) proteins, tau-1 and MAP-2. Neurons may also be identified based on the expression of any of a number of classical neurotransmitter enzymes. In addition, neurons may be identified based on morphological criteria and other functional measures such as electrophysiological recording. Such identification may be performed in combination with the identification of one or more of the phenotypic markers listed above.

Type I astrocytes are glial cells that have a flat protoplasmic/fibroblast-like morphology. These cells have the phenotype GFAP(+), A2B5 (-), GaIC(-), and MBP(-). Type II astrocytes, which are glial cells that display a stellate process-bearing morphology, have the phenotype GFAP(+), A2B5(+), GaIC(-), and MBP(-).

Once the cells have differentiated, they are again assayed for expression of the desired protein. Cells having the desired phenotype can be isolated and implanted into recipients in need of the protein or biologically active molecule that is expressed by the genetically modified cell.

Any suitable method for the implantation of precursor cells into the CNS may be used so that the cells appropriately integrate into the CNS. Injection methods exemplified by those used by Duncan et al. *J. Neurocytology*, 17:351-361 (1988), and scaled up and modified for use in humans are preferred. Methods taught by Gage et al. US Patent No. 5,082,670, for the injection of cell suspensions such as fibroblasts into the CNS may also be employed for injection of precursor cells. Additional approaches and methods may be found in *Neural Grafting in the Mammalian CNS,* Bjorklund and Stenevi, eds., (1985). The number of genetically modified cells injected will be that required to provide a therapeutic effect, and will vary depending upon the condition being treated. Generally, genetically modified neural precursor cells will be present in the brain at a concentration greater than 10 cells/100 cubic microns.

When the precursor cells are to be injected into the recipient and are derived from non-host tissue (i.e. allograft or xenograft), immunosuppression techniques may be required to insure longevity of the transplant. Local immunosuppression is disclosed by Gruber, *Transplantation* 54:1-11 (1992), incorporated by reference. Rossini, US Patent No. 5,026,365, discloses encapsulation methods suitable for local immunosuppression.

As an alternative to employing immunosuppression techniques, methods of gene replacement or knockout using homologous recombination in embryonic stem cells, taught by Smithies et al (Nature, 317:230-234 (1985), and extended to gene replacement or knockout in cell lines (H. Zheng 35 al., PNAS, 88:8067-8071 (1991)), can be applied to precursor cells for the ablation of major histocompatibility complex (MHC) genes. Precursor cells lacking MHC expression would allow for the grafting of enriched neural cell populations across allogeneic, and perhaps even xenogeneic, histocompatibility barriers without the need to immunosuppress the recipient. General reviews and citations for the use of recombinant methods to reduce antigenicity of donor cells are also disclosed by Gruber (*supra*). Exemplary approaches to the reduction of immunogenicity of transplants by surface modification are disclosed by Faustman WO 92/04033 (1992).

Although solid tissue fragments and cell suspensions of neural tissue are immunogenic as a whole, it could be possible that individual cell types within the graft are themselves immunogenic to a lesser degree. For example, Bartlett et al. (Prog. Brain Res. 82: 153-160 (1990)), have abrogated neural allograft rejection by pre-selecting a subpopulation of embryonic neuroepithelial cells for grafting by the use of immunobead separation on the basis of MHC expression. Thus, another approach is provided to reduce the chances of allo and xenograft rejection by the recipient without the use of immunosuppression techniques.

Results from human transplantation trials have demonstrated that fetal mesencephalic tissue allograft survival in the Parkinsonian brain is relatively poor (Freed et al and Spencer et al *supra*). The poor survival of the grafted material could be caused by the same disease-associated factors that resulted in the original neurodegeneration. One might predict that the environment, which was toxic to the recipient's own neurons, may be similarly toxic to the grafted tissue or cells. Support for this comes from reports of significant functional improvement provided by the implanted fetal mesencephalic tissue in MPTP-lesioned humans (Widner et al *supra*). Unlike the situation in the Parkinson's Disease brain, the MPTP-induced Parkinsonism is primarily a functional loss of dopaminergic neurons of the substantia nigra without the underlying idiopathic processes associated with Parkinson's Disease. Unlike the Parkinson's Disease brain, the MPTP-lesioned human brain is likely to be clear of neurotoxic substances at the time of the implant.

Recently, a novel chromaffin granule amine transporter (CGAT) cDNA has been described by Liu et al (Cell, 70:539-551 (1992)), which affords resistance to the neurotoxin MPP + in CHO cells *in vitro.* Because dopaminergic neurons from the substantia nigra are specifically killed by MPP+, CGAT gene expression in precursor cells may improve viability of the cells after they are implanted into the Parkinsonian brain.

In order that the invention described herein may be more fully understood, the following examples are set forth. It should be understood that these examples are for illustrative purposes only and are not to be construed as limiting the scope of the invention in any manner.

### EXAMPLES

### Example 1 Propagation of precursor cells

Embryonic day 14 (E14) CD₁ albino mice (Charles River) were decapitated and the brain and striata removed using sterile procedure. The tissue was mechanically dissociated with a fire-polished Pasteur pipette into serum-free medium composed of a 1:1 mixture of Dulbecco's modified Eagle's medium (DMEM) and F-1 2 nutrient mixture (Gibco). The cells were centrifuged at 800 r.p.m. for 5 minutes, the supernatant aspirated, and the cells resuspended in DMEM/F-12 medium for counting.

The cells were suspended in a serum-free medium, hereinafter referred to as "complete medium", composed of DMEM/F-12 (1:1) which included glucose (0.6%), glutamine (2 mM), sodium bicarbonate (3 mM), HEPES (4-[2-hydroxyethyl]-1-piperazineethanesulfonic acid) buffer (5 mM) and a defined hormone mix and salt mixture (to replace serum) that included insulin (25 µg/ml), transferrin (100 µg/ml), progesterone (20 nM), putrescine (60 µM), and selenium chloride (30 nM) (all from Sigma except glutamine [Gibco]). In addition, the medium contained 16-20 ng/ml EGF (purified from mouse submaxillary gland, Collaborative Research) or TGFα (human recombinant, Gibco). The cells were plated at 0.2 x 10⁶ cells/ml into 75 cm² tissue culture flasks (Corning) with no substrate pre-treatment and housed in an incubator at 37°C, 100% humidity, 95% air/5% CO₂.

The cells proliferated within the first 48 hours and by 3-4 days in vitro (DIV) formed small clusters, known as neurospheres, that lifted off the substrate between 4-6 DIV.

After 7 DIV, the neurospheres were removed, centrifuged at 400 r.p.m. for 2-5 minutes, and the pellet mechanically dissociated into individual cells with a fire-polished glass Pasteur pipet in 2 mls of complete medium.

1 x 10⁶ cells were replated into a 75 cm² tissue culture flask with 20 mls of the EGF-containing complete medium. The proliferation of the stem cells and formation of new neurospheres was reinitiated. This procedure can be repeated every 6-8 days to generate an unlimited number of stem cells.

### Example 2

### Genetic modification of precursor cells with a retrovirus containing the bacterial β-galactosidase gene

Precursor cells were propagated as described in Example 1. A large pass-1 flask of neurospheres (4-5 days old) was shaken to dislodge the spheres from the flask. The flask was spun at 400 r.p.m. for 3-5 minutes. About half of the media was removed without disturbing the neurospheres. The spheres and the remaining media were removed, placed into a Falcon 12 ml centrifuge tube, and spun at 600 r.p.m. for 3-5 minutes. The remaining medium was removed, leaving a few hundred microliters.

A retrovirus which contained the bacterial β-galactosidase gene was packaged and secreted, in a replication-deficient fashion, by the CRE BAG2 cell line produced by C. Cepko. A day after the CRE cells reached confluence, the cells were washed with PBS and the retrovirus was collected in DMEM/F12/HM/20 ng/ml EGF for four days. The virus-containing media was filtered through a .45 µm syringe filter. The neurospheres were resuspended in the virus-containing media, transferred to a large flask, and left in an incubator overnight at 37° C. The next day, the contents of the flask were transferred to a 12 ml centrifuge tube and spun at 800 r.p.m. The cells were resuspended in EGF-containing media/HM, dissociated into single cells, and counted. The cells were replated in a large flask at 50,000 cells/ml in a total of 20 mls.

Four days later, transformed cells were selected with G418 at a concentration of 300 µg/ml. Transformed spheres were plated on a polyornithine coated glass coverslip in a 24-well plate. After the neurospheres adhered to the plate, the cells were fixed with 0.1 % glutaraldehyde for 5 minutes at 4°C. After the cells were fixed, they were washed twice with PBS for 10 minutes. The cells were then washed with 0.1 % Triton^{R} in PBS for 10-15 minutes at room temperature. A 1 mg/ml X-Gal solution was added to each well and incubated overnight at 37°C. After incubation overnight, the cells were washed three times with PBS for 10 minutes each and observed for any reaction products. A positive reaction resulted in a blue color, indicating cells containing the transferred gene.

### Example 3

### Preparation of precursor cells from transgenic mice

Transgenic mice were produced using standard pronuclear injection of the MBP*-lacZ* chimeric gene, in which the promoter for MBP directs the expression of *E. coli* β-galactosidase (*lacZ*). Transgenic animals were identified by PCR using oligonucleotides specific for *lacZ*.

Neurospheres were prepared from E15 transgenic mice and DNA negative littermates using the procedures set forth in Example 1. The neurospheres were propagated in the defined culture medium in the presence of 20 ng/ml EGF and were passaged weekly for 35 weeks. For passaging, the neurospheres were harvested, gently centrifuged at 800 RPM, and mechanically dissociated by trituration with a fire-polished Pasteur pipet. At various passages, the cells were induced to differentiate into oligodendrocytes, astrocytes, and neurons by altering the culture conditions. The free-floating stem cell clusters were gently centrifuged, resuspended in the same base defined medium without EGF and with 1 % fetal bovine serum and plated on poly ornithine-treated glass coverslips to promote cell attachment. The clusters attach firmly to the glass, and the cells slow or stop dividing and begin to differentiate.

The identification of various cell types was accomplished using immunofluorescence microscopy with antibodies specific for neurons (MAP-2, NF-L, and NF-M), astrocytes (GFAP) and oligodendrocytes and oligodendrocyte precursors (A2B5, O₁, O₄, Gal C, and MBP). One to 14 days post-plating, the cells on the coverslips were incubated unfixed, for 30 minutes at room temperature with the primary antibodies O1, O4, GaIC, and A2B5 (supernatants) diluted in minimal essential medium with 5% normal goat serum and 25 mM HEPES buffer, pH 7.3 (MEM-HEPES, NGS). Following the primary antibodies, the coverslips were gently washed 5 times in rhodamine-conjugated secondary antibodies (Sigma) diluted in MEM-HEPES, NGS. The coverslips were then washed 5 times in MEM-HEPES and fixed with acid alcohol (5% glacial acetic acid/95% ethanol) at -20° C. The coverslips were then washed 5 times with MEM-HEPES, and either mounted and examined using fluorescence microscopy or immmunoreacted with rabbit polyclonal antisera raised against GFAP, nestin, MBP, or proteolipid protein (PLP). When subjected to a second round of immunotabeling, the coverslips were incubated first for 1 hour with 5% normal goat serum (NGS) in 0.1 M phosphate buffer with 0.9% NaCl at pH 7.4 (PBS) and then incubated in rabbit primary antibodies diluted in NGS for 1-2 hours at room temperature. The coverslips were washed 3 times with PBS and then incubated with the appropriate secondary antibody conjugates diluted in NGS, washed again with PBS and then finally mounted on glass microscope slides with Citifluor antifadent mounting medium and examined using a fluorescence microscope. In cases were they were not incubated first with the monoclonal antibody supernatants, the coverslips were fixed for 20 minutes with 4% paraformaldehyde in PBS (pH 7.4), washed with PBS, permeabilized with 100% ethanol, washed again with PBS and incubated with 5% NGS in PBS for 1 hour. The primary antibodies and secondary antibody conjugates were applied as outlined above.

The neural stem cells derived from the transgenic animals were indistinguishable from non transgenic stem cells in their potential for differentiation into neurons, astrocytes, and oligodendrocytes. The MBP promoter directed the expression of the β-galactosidase reporter gene in a cell-specific and developmentally appropriate fashion. The transgene expression is highly stable as oligodendrocytes derived from late passage MBP-*lacZ* neurospheres (20 passages), expressed the β-galactosidase gene. Thus, transgenically marked neurospheres are likely to be an excellent source of cells for glial cell transplantation.

### Example 4

### Genetic modification of precursor cells using calcium phosphate transfection

Precursor cells are propagated as described in Example 1. The cells are then infected using a calcium phosphate transfection technique. For standard calcium phosphate transfection, the cells are mechanically dissociated into a single cell suspension and plated on tissue culture-treated dishes at 50% confluence (50,000-75,000 cells/cm²) and allowed to attach overnight.

The modified calcium phosphate transfection procedure is performed as follows: DNA (15-25 µg) in sterile TE buffer (10 mM Tris, 0.25 mM EDTA, pH 7.5) diluted to 440 µl with TE, and 60 µl of 2 M CaCl₂ (pH to 5.8 with 1 M HEPES buffer) is added to the DNA/TE buffer. A total of 500 µl of 2 x HeBS (HEPES-Buffered saline; 275 mM NaCl, 10 mM KCI, 1.4 mM Na₂HPO₄, 12 mM dextrose, 40 mM HEPES buffer powder, pH 6.92) is added dropwise to this mix. The mixture is allowed to stand at room temperature for 20 minutes. The cells are washed briefly with 1 x HeBS and 1 ml of the calcium phosphate precipitated DNA solution is added to each plate, and the cells are incubated at 37° for 20 minutes. Following this incubation, 10 mls of complete medium is added to the cells, and the plates are placed in an incubator (37°C, 9.5% CO₂) for an additional 3-6 hours. The DNA and the medium are removed by aspiration at the end of the incubation period, and the cells are washed 3 times with complete growth medium and then returned to the incubator.

### Example 5

### Genetically modified precursor cells expressing NGF

Using either the recombinant retrovirus or direct DNA transfection technique, a chimeric gene composed of the human cytomegalovirus CMV promoter directing the expression of the rat NGF gene is introduced into the neurosphere cells. In addition, the vector includes the *E. coli* neomycin resistance gene driven off of a viral promoter. After 2 weeks of G418 selection, the cells are cloned using limiting dilution in 96-multi-well plates and the resulting clones are assayed for neurotrophin protein expression using a neurotrophin receptor (*trk* family) autophosphorylation bioassay.

Clones expressing high levels of NGF are expanded in T-flasks prior to differentiation. The cells are then removed from the EGF-containing complete medium and treated with a combination of serum and a cocktail of growth factors to induce astrocyte differentiation. The astrocytes are again assayed for NGF expression to ensure that the differentiated cells continue to express the trophic factors. Astrocytes that secrete NGF are then injected into fimbria/fornix lesioned rat brains immediately post-lesioning in order to protect the cholinergic neurons. Control astrocytes that do not secrete NGF are injected into similarly lesioned animals. The sparing of cholinergic neurons in the lesion model is assessed using immunocytochemistry for ChAT, the marker for these cholinergic neurons.

### Example 6

### Genetically modified precursor cells expressing CGAT

Precursor cells are propagated as in Example 1. The cells are mechanically dissociated and plated on plastic dishes and infected with a retrovirus containing the CGAT cDNA. The expression of the CGAT cDNA (Liu et al *supra*) is directed by a constitutive promoter (CMV or SV40, or a retroviral LTR) or a cell-specific promoter (TH or other dopaminergic or catecholaminergic cell-specific regulatory element or the like). The cells are screened for the expression of the CGAT protein.

Selected cells can then be differentiated *in vitro* using a growth factor or a combination of growth factors to produce dopaminergic or pre-dopaminergic neurons.

## Claims

1. A method of producing non-tumorigenic, genetically modified multipotent central nervous system (CNS) neural stem cells, comprising the steps of:
(a) isolating multipotent CNS neural stem cells from neural tissue to the exclusion of human embryonic tissue/cells, wherein the neural stem cells have not been immortalised with an oncogene, said CNS neural stem cells being capable of producing progeny that are capable of differentiating into neurons,' astrocytes, and oligodendrocytes;
(b) proliferating said multipotent CNS neural stem cells in a serum-free culture medium containing a growth factor effective for inducing multipotent CNS neural stem cell proliferation,
(c) further propagating the proliferated cells, and
(d) genetically modifying said propagated cells, by introducing an exogenous polynucleotide to the proliferated cells.

2. The method of claim 1, wherein the growth factor effective for inducing multipotent CNS neural stem cell proliferation in the culture medium in step (b) is selected from the group consisting of acidic fibroblast growth factor, basic fibroblast growth factor, epidermal growth factor, amphiregulin, transforming growth factor alpha, and combinations' thereof.

3. The method of claim 1 or claim 2, wherein said neural tissue is obtained from a foetus.

4. The method of claim 1 or claim 2, wherein said neural tissue is obtained from an adult.

5. The method of any one of claims 1, 2 or 4, wherein said neural tissue is obtained from a human.

6. The method of any preceding claim, wherein the proliferated cells are genetically modified to produce a growth factor product.

7. The method of claim 6, wherein the growth factor product is selected from the group consisting of nerve growth factor, brain-derived neurotrophic factor, neurotrophins, ciliary neurotrophic factor', amphiregulin, basic fibroblast growth factor, acidic fibroblast growth factor, epidermal growth factor, transforming growth factor-alpha, transforming growth factor-beta, platelet-derived growth factor, insulin-like growth factors, and interleukins.

8. The method of any one of claims 1 to 6, wherein the proliferated cells are genetically modified to produce a growth factor receptor.

9. The method of claim 8, wherein the growth factor receptor is selected from the group consisting of p75 low affinity nerve growth factor receptor, ciliary neurotrophic factor receptor, neurotrophin receptors, epidermal growth factor receptor, fibroblast growth factor receptor, and amphiregulin receptor.

10. The method of any one of claims 1 to 6, wherein the proliferated cells are genetically modified to express a neurotransmitter.

11. The method of claim 10, wherein the neurotransmitter is selected from the group consisting of serotonin, L-dopa, dopamine, norepinephrine, epinephrine, tachykinin, endorphin, histamine, N-methyl-D-aspartate, glycine, glutamate, γ-amino butyric acid, and acetylcholine.

12. The method of any one of claims 1 to 6, wherein the proliferated cells are genetically modified to contain a neurotransmitter synthesizing gene.

13. The method of claim 12, wherein the neurotransmitter synthesizing gene is selected from the group consisting of tyrosine hydroxylase, dopa decarboxylase, dopamine-β-hydroxylase, phenylethanolamine N-methyltransferase, glutamic acid decarboxylase, tryptophan hydroxylase, choline acetyltransferase, and histidine decarboxylase.

14. The method of.any one of claims 1 to 6, wherein the proliferated cells are genetically modified to produce a neuropeptide.

15. The method of claim 14, wherein the neuropeptide is selected from the group consisting of substance-P, neuropeptide-y, enkephalin, vasopressin, vasoactive intestinal peptide, cholecystokinin, glucagon, bombesin, somatostatin, and calcitonin gene-related peptide.

16. The method of any one of claims 1 to 6, wherein the proliferated cells are genetically modified to express chromaffin granule amine transporter.

17. The method of any preceding claim, further comprising:
(d) differentiating the genetically modified cell.

18. A culture comprising genetically modified multipotent central nervous system (CNS) neural stem cells which have not been immortalised with an oncogene, and which:
(a) stain positively for nestin;
(b) are capable of producing progeny that are capable of differentiation to oligodendrocytes, astrocytes, and neurons; and
(c) are non-tumorigenic,
wherein said cell culture is obtainable by the steps of:
(a) isolating multipotent CNS neural stem cells from neural tissue, wherein the neural stem cells have not been immortalised with an oncogene, said CNS neural stem cells being capable of producing progeny that are capable of differentiating into neurons, astrocytes, and oligodendrocytes;
(b) proliferating said multipotent CNS neural stem cells in a serum-free culture medium containing a growth factor effective for inducing multipotent CNS neural stem cell proliferation,
(c) further propagating the proliferated cells, and
(d) genetically modifying said propagated cells, by introducing an exogenous polynucleotide to the proliferated cells.

19. The culture of claim 18, wherein the cell is a human cell.

20. The culture of claim 19 or 19, wherein the cells are genetically modified to express a biologically active substance selected from the group consisting of growth factor products, growth factor receptors, neurotransmitters, neurotransmitter receptors and neuropeptides.

## Patentansprüche

1. Verfahren zur Herstellung von nicht tumorigenen, genetisch veränderten, multipotenten neuralen Stammzellen des zentralen Nervensystems (ZNS), umfassend die Schritte:
(a) Isolieren von multipotenten neuralen Stammzellen des ZNS aus neuralem Gewebe unter Ausschluss von humanem Embryonalgewebe/Embryonalzellen, wobei die neuralen Stammzellen nicht mit einem Oncogen immortalisiert wurden, wobei die neuralen Stammzellen des ZNS Nachkommen erzeugen können, die sich in Neuronen, Astrozyten und Oligodendrozyten differenzieren können;
(b) Proliferieren der multipotenten neuralen Stammzellen des ZNS in einem serumfreien Kulturmedium, das einen Wachstumsfaktor enthält, welcher eine Induktion der Proliferation der multipotenten neuralen Stammzellen des ZNS bewirken kann;
(c) weiteres Vermehren der proliferierten Zellen, und
(d) genetisches Verändern der vermehrten Zellen durch Einbringen eines exogenen Polynukleotids in die proliferierten Zellen.

2. Verfahren nach Anspruch 1, worin der Wachstumsfaktor, welcher eine Induktion der Proliferation der multipotenten neuralen Stammzellen des ZNS im Kulturmedium in Schritt (b) bewirken kann, ausgewählt wird aus der Gruppe bestehend aus saurem Fibroblastenwachstumsfaktor, basischem Fibroblastenwachstumsfaktor, epidermalem Wachstumsfaktor, Amphiregulin, transformierendem Wachstumsfaktor Alpha und Kombinationen davon.

3. Verfahren nach Anspruch 1 oder Anspruch 2, worin das neurale Gewebe von einem Foetus erhalten wird.

4. Verfahren nach Anspruch 1 oder Anspruch 2, worin das neurale Gewebe von einem erwachsenen Lebewesen erhalten wird.

5. Verfahren nach einem der Ansprüche 1, 2 oder 4, worin das neurale Gewebe von einem Menschen erhalten wird.

6. Verfahren nach einem der vorhergehenden Ansprüche, worin die proliferierten Zellen genetisch verändert werden, um ein Wachstumsfaktorprodukt zu erzeugen.

7. Verfahren nach Anspruch 6, worin das Wachstumsfaktorprodukt ausgewählt wird aus der Gruppe bestehend aus Nervenwachstumsfaktor, hirnstämmigem neurotrophem Faktor ("brain-derived neurotrophic factor"), Neurotrophinen, ziliärem neurotrophem Faktor, Amphiregulin, basischem Fibroblastenwachstumsfaktor, saurem Fibroblastenwachstumsfaktor, epidermalem Wachstumsfaktor, transformierendem Wachstumsfaktor Alpha, transformierendem Wachstumsfaktor Beta, Plättchenwachstumsfaktor, Insulinähnlichen Wachstumsfaktoren und Interleukinen.

8. Verfahren nach einem der Ansprüche 1 bis 6, worin die proliferierten Zellen genetisch verändert werden, um einen Wachstumsfaktor-Rezeptor zu produzieren.

9. Verfahren nach Anspruch 8, worin der Wachstumsfaktor-Rezeptor ausgewählt wird aus der Gruppe bestehend aus dem Rezeptor des p75 Nervenwachstumsfaktors mit geringer Affinität, dem Rezeptor des ziliären neurotrophen Faktors, Neurotrophinrezeptoren, dem Rezeptor des epidermalen Wachstumsfaktors, dem Rezeptor des Fibroblastenwachstumsfaktors und dem Amphiregulinrezeptor.

10. Verfahren nach einem der Ansprüche 1 bis 6, worin die proliferierten Zellen genetisch verändert werden, um einen Neurotransmitter zu exprimieren.

11. Verfahren nach Anspruch 10, worin der Neurotransmitter ausgewählt wird aus der Gruppe bestehend aus Serotonin, L-Dopa, Dopamin, Norepinephrin, Epinephrin, Tachykinin, Endorphin, Histamin, N-Methyl-D-Aspartat, Glycin, Glutamat, γ-Aminobuttersäure und Acetylcholin.

12. Verfahren nach einem der Ansprüche 1 bis 6, worin die proliferierten Zellen genetisch verändert werden, um ein Neurotransmitter-bildendes Gen zu enthalten.

13. Verfahren nach Anspruch 12, worin das Neurotransmitter-bildende Gen ausgewählt wird aus der Gruppe bestehend aus Tyrosin-Hydroxylase, Dopa-Decarboxylase, Dopamin-β-Hydroxylase, Phenylethanolamin-N-Methyltransferase, Glutaminsäure-Decarboxylase, Tryptophan-Hydroxylase, Cholin-Acetyltransferase und Histidin-Decarboxylase.

14. Verfahren nach einem der Ansprüche 1 bis 6, worin die proliferierten Zellen genetisch verändert werden, um ein Neuropeptid zu produzieren.

15. Verfahren nach Anspruch 14, worin das Neuropeptid ausgewählt wird aus der Gruppe bestehend aus der Substanz P, Neuropeptid-γ, Enkephalin, Vasopressin, vasoaktivem intestinalem Peptid, Cholecystokinin, Glucagon, Bombesin, Somatostatin und Calcitoningen-verwandtem Peptid.

16. Verfahren nach einem der Ansprüche 1 bis 6, worin die proliferierten Zellen genetisch verändert werden, um den Chromaffin-Granulum-Amintransporter zu exprimieren.

17. Verfahren nach einem der vorhergehenden Ansprüche, weiter umfassend:
(d) Differenzieren der genetisch veränderten Zelle.

18. Kultur, umfassend genetisch veränderte, multipotente, neurale Stammzellen des zentralen Nervensystems (ZNS), welche nicht mit einem Onkogen immortalisiert wurden, und welche
(a) eine positive Nestin-Färbung aufweisen;
(b) Nachkommen erzeugen können, welche sich zu Oligodendrozyten, Astrozyten und Neuronen differenzieren können, und
(c) nicht tumorigen sind,
worin die Zellkultur erhältlich ist durch die Schritte:
(a) Isolieren von multipotenten neuralen Stammzellen des ZNS aus neuralem Gewebe, worin die neuralen Stammzellen nicht mit einem Oncogen immortalisiert wurden, wobei die neuralen Stammzellen des ZNS Nachkommen erzeugen können, die sich in Neuronen, Astrozyten und Oligodendrozyten differenzieren können;
(b) Proliferieren der multipotenten neuralen Stammzellen des ZNS in einem serumfreien Kulturmedium, das einen Wachstumsfaktor enthält, welcher eine Induktion der Proliferation der multipotenten neuralen Stammzellen des ZNS bewirken kann;
(c) weiteres Vermehren der proliferierten Zellen, und
(d) genetisches Verändern der vermehrten Zellen durch Einbringen eines exogenen Polynukleotids in die proliferierten Zellen.

19. Kultur nach Anspruch 18, worin die Zelle eine humane Zelle ist.

20. Kultur nach Anspruch 18 oder 19, worin die Zellen genetisch verändert werden, um eine biologisch aktive Substanz zu exprimieren, welche ausgewählt wird aus der Gruppe bestehend aus Wachstumsfaktorprodukten, Wachstumsfaktor-Rezeptören, Neurotransmittern, Neurtransmitter-Rezeptoren und Neuropeptiden.

## Revendications

1. Procédé de production de cellules souches neuronales du système nerveux central (SNC) totipotentes, non tumorigènes et génétiquement modifiées, comprenant les étapes :
(a) d'isolement de cellules souches neuronales totipotentes du SNC à partir d'un tissu neuronal, à l'exclusion de tissu/cellules embryonnaires humaines, les cellules souches neuronales n'ayant pas été immortalisées avec un oncogène, lesdites cellules souches neuronales du SNC étant capables de générer une descendance qui est capable de se différencier en neurones, en astrocytes et en oligodendrocytes ;
(b) de prolifération desdites cellules souches neuronales totipotentes du SNC dans un milieu de culture sans sérum contenant un facteur de croissance efficace pour induire la prolifération de cellules souches neuronales totipotentes du SNC,
(c) de propagation additionnelle des cellules ayant proliféré et
(d) de modification génétique desdites cellules propagées pur introduction d'un polynucléotide exogène dans les cellules ayant proliféré.

2. Procédé selon la revendication 1, dans lequel le facteur de croissance efficace pour induire la prolifération de cellules souches neuronales totipotentes du SNC dans le milieu de culture de l'étape (b) est choisi dans le groupe formé par le facteur acide de croissance des fibroblastes, le facteur basique de croissance des fibroblastes, le facteur de croissance épidermique, l'amphiréguline, le facteur de croissance transformant alpha et une combinaison de ceux-ci.

3. Procédé selon la revendication 1 ou 2, dans lequel ledit tissu neuronal est obtenu à partir d'un foetus.

4. Procédé selon la revendication 1 ou 2, dans lequel ledit tissu neuronal est obtenu à partir d'un adulte.

5. Procédé selon l'une quelconque des revendications 1, 2 ou 4, dans lequel ledit tissu neuronal est obtenu à partir d'un être humain.

6. Procédé selon l'une quelconque des revendications précédentes, dans lequel les cellules ayant proliféré sont génétiquement modifiées pour produire un facteur de croissance.

7. Procédé selon la revendication 6, dans lequel le facteur de croissance produit est choisi dans le groupe formé par le facteur de croissance des neurones, le facteur neurotrophique dérivé du cerveau, les neurotrophines, le facteur ciliaire neurotrophique, l'amphiréguline, le facteur basique de croissance des fibroblastes, le facteur acide de croissance des fibroblastes, le facteur de croissance épidermique, le facteur de croissance transformant alpha, le facteur de croissance transformant bêta, le facteur de croissance dérivé des plaquettes, les facteurs de croissance du type insulinique et les interleukines.

8. Procédé selon l'une quelconque des revendications 1 à 6, dans lequel les cellules ayant proliféré sont génétiquement modifiées pour produire un récepteur d'un facteur de croissance.

9. Procédé selon la revendication 8, dans lequel le récepteur d'un facteur de croissance est choisi dans le groupe formé par le récepteur du facteur de croissance des neurones de basse affinité p75, le récepteur du facteur ciliaire neurotrophique, les récepteurs neurotrophiques, le récepteur du facteur de croissance épidermique, le récepteur du facteur de croissance des fibroblastes et le récepteur d'amphiréguline.

10. Procédé selon l'une quelconque des revendications 1 à 6, dans lequel les cellules ayant proliféré sont génétiquement modifiées pour exprimer un neurotransmetteur.

11. Procédé selon la revendication 10, dans lequel le neurotransmetteur est choisi dans le groupe formé par la sérotonine, la L-dopa, la dopamine, la norépinéphrine, l'épinéphrine, la tachykinine, une endorphine, une histamine, le N-méthyl-D-aspartate, la glycine, le glutamate, l'acide γ-aminobutyrique et l'acétylcholine.

12. Procédé selon l'une quelconque des revendications 1 à 6, dans lequel les cellules ayant proliféré sont génétiquement modifiées pour contenir un gène synthétisant un neurotransmetteur.

13. Procédé selon la revendication 12, dans lequel le gène synthétisant un neurotransmetteur est choisi dans le groupe formé par la tyrosine hydroxylase, la dopa décarboxylase, la dopamine-β-hydroxylase, la phényléthanolamine N-méthyltransférase, la glutamate décarboxylase, la tryptophane hydroxylase, la choline acétyltransférase et l'histidine décarboxylase.

14. Procédé selon l'une quelconque des revendications 1 à 6, dans lequel les cellules ayant proliféré sont génétiquement modifiées pour produire un neuropeptide.

15. Procédé selon la revendication 14, dans lequel le neuropeptide est choisi dans le groupe formé par la substance-P, le neuropeptide-γ, l'enképhaline, la vasopressine, le peptide intestinal vasomoteur, la cholécystokinine, le glucagon, la bombésine, la somatostatine et un peptide apparenté au gène de la calcitonine.

16. Procédé selon l'une quelconque des revendications 1 à 6, dans lequel les cellules ayant proliféré sont génétiquement modifiées pour exprimer un transporteur d'amines de granules de chromaffine.

17. Procédé selon l'une quelconque des revendications précédentes, comprenant en outre :
(d) la différenciation de la cellule génétiquement modifiée.

18. Culture comprenant des cellules souches neuronales du système nerveux central (SNC) totipotentes et génétiquement modifier qui n'ont pas été immortalisées avec un oncogène et qui :
(a) se colorent positivement à la nestine ;
(b) sont capables de générer une descendance qui est capable de se différencier en oligodendrocytes, en astrocytes et en neurones ; et
(c) ne sont pas tumorigènes,
dans laquelle ladite culture cellulaire peut être obtenue par les étapes :
(a) d'isolement de cellules souches neuronales totipotentes du SNC à partir d'un tissu neuronal dans lequel les cellules souches neuronales n'ont pas été immortalisées avec un oncogène, lesdites cellules souches neuronales du SNC étant capables de générer une descendance qui est capable de se différencier en neurones, en astrocytes et en oligodendrocytes ;
(b) de prolifération desdites cellules souches neuronales totipotentes du SNC dans un milieu de culture sans sérum contenant un facteur de croissance efficace pour induire une prolifération de cellules souches neuronales totipotentes du SNC,
(c) de propagation additionnelle des cellules ayant proliféré et
(d) de modification génétique desdites cellules propagées par introduction d'un polynucléotide exogène dans les cellules ayant proliféré.

19. Culture selon la revendication 18, dans laquelle la cellule est une cellule humaine.

20. Culture selon la revendication 18 ou 19, dans laquelle les cellules sont génétiquement modifiées pour exprimer une substance biologiquement active choisie dans le groupe composé de facteurs de croissance, de récepteurs de facteurs de croissance, de neurotransmetteurs, de récepteurs de neurotransmetteurs et de neuropeptides.
